# EUROPÄISCHE PATENTSCHRIFT

(11) **EP 0 577 949 B1**
(45) Veröffentlichungstag und Bekanntmachung des Hinweises auf die Patenterteilung: **14.08.1996**
(21) Anmeldenummer: 93107523.8
(22) Anmeldetag: 08.05.1993
(51) Int. Cl.: C07C 67/317, C07C 69/74

(54) **Verfahren zur Herstellung von chlorfreiem Cyclopropancarbonsäuremethylester**
Method of preparing chlorine free cyclopropanecarbocylicacid methylester
Procédé pour la préparation d'ester de méthyle d'acide cyclopropane carboxylique exempt de chlore

(30) Priorität: 09.07.1992 DE 4222497
(43) Veröffentlichungstag der Anmeldung: 12.01.1994
(73) Patentinhaber: HÜLS AKTIENGESELLSCHAFT, D-45764 Marl (DE)
(72) Erfinder: Kaufhold, Manfred, Dr., W-4370 Marl (DE); Metz, Josef, Dr., W-4370 Marl (DE)

(56) Entgegenhaltungen:
- DE-A- 1 939 759
- GB-A- 2 008 110

## Beschreibung

Die Erfindung betrifft ein Verfahren zur Herstellung von reinem, chlorfreiem Cyclopropancarbonsäuremethylester der Formel 1 durch Umsetzung von 4-Chlorbuttersäuremethylester (2) mit Natriummethylat in einem hochsiedenden Lösungsmittel bei 100 bis 200 °C unter gleichzeitigem Abdestillieren des entstandenen Cyclopropancarbonsäuremethylesters und anschließender fraktionierter Reindestillation dieses Esters.

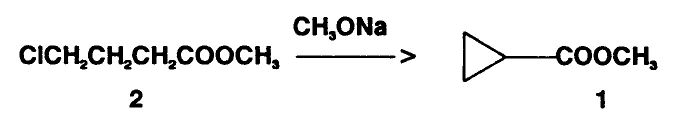

Synthesen von Cyclopropancarbonsäuremethylester aus 4-Chlorbuttersäuremethylester und stark basischen Substanzen sind literaturbekannt.

In US-PS 3 294 833 wird Natriumamid als basische Substanz vorgeschlagen, während in DE-AS 19 39 759 und DE-OS 27 51 133 Natriummethylat in Toluol empfohlen wird.

Bei der Durchführung dieser Verfahren wird das basische Mittel mit einem inerten Lösungsmittel gemischt, die Mischung erwärmt und der 4-Chlorbuttersäuremethylester zugegeben. Hierbei muß das gebildete Methanol als Azeotrop abdestilliert werden, da sonst infolge des niedrigen Siedepunktes des Methanols die Reaktionstemperatur sinkt und die Reaktionszeit dadurch verlängert wird. Im Falle des Natriumamids entsteht ein Ammoniak-Gasstrom, der entsorgt werden muß.

Diese Verfahrensweisen arbeiten also mit Suspensionen, die zu Verklebungen und Anbackungen neigen und dadurch bedingt viele technische Probleme mit sich bringen. Wenn festes Alkoholat in der Apparatur anfällt, besteht wegen der hohen Temperatur außerdem Explosionsgefahr. Deshalb muß in starker Verdünnung gearbeitet werden, wodurch aber die Raum-Zeit-Ausbeute verschlechtert wird.

Deshalb wird bei dem Verfahren der DE-PS 29 41 211 ohne inertes Lösungsmittel mit einer Lösung von Natriummethylat in Methanol gearbeitet, und zwar entweder bei hohen Temperaturen, z. B. 155 bis 160 °C, unter Druck oder bei tieferen Temperaturen, z. B. 90 bis 100 °C, unter solchen exakten geregelten Bedingungen, bei denen die jeweils richtige Methanolmenge abdestilliert wird oder mit Hilfe eines speziellen Reaktionsverdampfers, der ebenfalls sehr exakt gesteuert werden muß. Die Aufarbeitung der Reaktionsprodukte ist aufwendig, erfordert eine Extraktion mit Methylenchlorid und führt zu einem mit Methanol verunreinigten Abwasser. Nachteilig ist weiterhin die Verwendung von speziellen Reaktoren.

Ein gravierender Nachteil bei dem Verfahren der DE-PS 29 41 211 ist zudem die zu geringe Reinheit der erhaltenen Cyclopropancarbonsäureester. Denn der Chlorgehalt dieser Ester liegt im allgemeinen bei 100 bis 1 000 ppm oder höher. Wegen dieser hohen Chlorgehalte können diese Ester nicht katalytisch zum Cyclopropylcarbinol hydriert werden.
Bei den Reaktoren würde Korrosion auftreten und der Hydrierkontakt würde vergiftet. Deshalb muß der Chlorgehalt der für katalytische Hydrierungen vorgesehenen Ester unter 10 ppm liegen.

Für die Reduktion der chlorhaltigen Cyclopropancarbonsäureester kommen also nur kostspielige Reagenzien, wie z. B. Lithiumaluminiumhydrid, in Frage.

Die EP-A 0 220 412 beschreibt ein Verfahren zur Herstellung von reinen, chlorfreien Cyclopropancarbonsäureestern, die als Ausgangsmaterial für katalytische Hydrierungen geeignet sind. Nachteilig bei diesem Verfahren ist, daß zunächst der Butylester entsteht, der in einer zusätzlichen Reaktionsstufe mit einem höhersiedenden Alkohol mit mehr als 4 C-Atomen umgeestert werden muß. Beim Einsatz des Butylesters in die Hydrierung würde ein Hydrieraustrag anfallen, der nur mit hohem Destillationsaufwand gereinigt werden kann.

Der Einsatz des Methylesters als Ausgangsprodukt für die Hydrierung hat grundsätzlich gegenüber den in der EP-A 0 220 412 hergestellten Estern den Vorteil, daß das Cyclopropylcarbinol mit wesentlich höherer Raum-Zeit-Ausbeute herstellbar ist. Denn die in äquimolaren Mengen entstehenden Alkohole verbrauchen sozusagen im Vergleich zum Methanol wertvollen Reaktionsraum.

Alle bekannten Verfahren benötigen teure Chemikalien, Druckapparaturen und führen zu Problemen mit der Abfallbeseitigung oder benötigen zusätzliche Syntheseschritte und verlaufen mit zu geringer Raum-Zeit-Ausbeute. Wünschenswert ist daher ein Verfahren, bei dem 4-Chlorbuttersäureester mittels Natriummethylat in üblichen Rührapparaturen ohne spezielle Regelungen drucklos zum chlorfreien Cyclopropancarbonsäureester umgesetzt wird. Der entstandene Cyclopropancarbonsäuremethylester kann beispielsweise katalytisch zum Cyclopropylcarbinol hydriert werden.

Es besteht ein großes Interesse an einem Verfahren, nach dem man bei geringem technischen Aufwand und ohne den Einsatz von teuren Reagenzien aus 4-Chlorbuttersäuremethylester Cyclopropancarbonsäureester und aus diesem gegebenenfalls Cyclopropylcarbinol herstellen kann. Diese Produkte sind wichtige Rohstoffe für Pharma-Produkte, und es war Aufgabe der Erfindung, ein derartiges Verfahren zu entwickeln.

Überraschenderweise erhält man in Lösung der Aufgabe chlorfreien Cyclopropancarbonsäuremethylester aus 4-Chlorbuttersäuremethylester, wenn man folgende Reaktionsbedingungen wählt:
1. ein hochsiedendes Lösungsmittel wird vorgelegt und auf beispielsweise 120 bis 130 °C erwärmt
2. eine bestimmte Teilmenge an einzusetzendem Natriummethylat wird sozusagen als Vorschuß unter Rühren zugegeben und dadurch eine rührfähige, dünnflüssige Suspension erzeugt
3. dann werden äquimolare Mengen an Natriummethylat und Chlorbuttersäuremethylester gleichzeitig zugegeben, gegebenenfalls restlicher Chlorbuttersäuremethylester und dabei der gebildete Cyclopropancarbonsäuremethylester gemeinsam mit Methanol abdestilliert
4. schließlich wird dieses Methanol-Ester-Gemisch fraktioniert reindestilliert.

Gegenstand der Erfindung ist daher ein Verfahren zur Herstellung von chlorfreiem Cyclopropancarbonsäuremethylester durch Umsetzung von 4-Chlorbuttersäuremethylester mit Natriummethylat in einem inerten Lösungsmittel, welches dadurch gekennzeichnet ist, daß man
a) zunächst das Lösungsmittel vorlegt, auf 100 bis 200 °C erwärmt,
b) einen Teil des einzusetzenden Alkalimethylats zugibt,
c) erst danach weiteres Alkalimethylat und 4-Chlorbuttersäuremethylester gleichzeitig in stöchiometrischem Verhältnis zugibt,
d) wobei ständig ein Gemisch von Methanol und Cyclopropancarbonsäuremethylester abdestilliert und
e) anschließend dieses Gemisch durch fraktionierte Destillation reinigt.

Es ist überraschend, daß der anfallende Ester nur dann chlorfrei und in guten Ausbeuten von über 85 % anfällt, wenn man die ganze Kombination der Einzelschritte durchführt. Vor der Reindestillation hat der rohe Ester noch hohe Chlorgehalte von 100 bis 500 ppm, d. h. der Grund für den resultierenden niedrigen Chlorgehalt liegt nicht in einer quantitativen Umsetzung. Verzichtet man beispielsweise auf die Durchführung der zweiten Stufe, so zeigen die Chlorgehalte nach der Reindestillation von Versuch zu Versuch starke Schwankungen. Arbeitet man sogar mit einem "Vorschuß" an Chlorbuttersäureester, dann fällt bei der Reindestillation ein chlorhaltiger Ester mit Chlorgehalten von 50 bis 200 ppm an.

Ein Vorteil dieses Verfahrens ist auch die einfache Aufarbeitung des Reaktionssumpfes, der nur aus hochsiedendem Lösungsmittel und Kochsalz besteht. Das Kochsalz kann abfiltriert oder mit Wasser ausgewaschen werden, und das Lösungsmittel kann wiederverwendet werden.

Die praktische Durchführung der einzelnen Stufen erfolgt beispielsweise in folgender Weise:
Man verwendet eine Rührapparatur ohne Destillationskolonne, nur mit einer Destillationsbrücke ausgerüstet, und legt ein hochsiedendes Lösungsmittel vor. Der Siedepunkt des Lösungsmittels soll über 150 °C, vorzugsweise über 250 °C, insbesondere über 280 °C, liegen. Das Lösungsmittel muß gegen Natriummethylat inert sein, braucht aber außer thermischer Stabilität keine weiteren Bedingungen zu erfüllen.

Aus wirtschaftlichen Gründen wählt man einen billigen Kohlenwasserstoff, wie z. B. einen Alkylaromaten bzw. ein Gemisch von Alkylaromaten, z. B. n-C₁₀-C₁₃-Alkylbenzol, Tetrapropylbenzol usw.

Das Lösungsmittel wird auf 100 bis 200 °C, vorzugsweise 100 bis 150 °C, insbesondere auf 120 bis 140 °C erwärmt und gerührt. Dann wird eine Teilmenge der einzusetzenden Menge des Natriummethylats in fester Form oder als methanolische Lösung zugegeben, wobei in der zuletzt genannten Form Methanol abdestilliert. Diese Teilmenge ist 50 bis 5 % der Gesamtmenge, vorzugsweise 10 bis 30 %, insbesondere 20 bis 25 %.
Direkt anschließend wird Natriummethylat und 4-Chlorbuttersäuremethylester in äquimolaren Mengen zugegeben, so daß der Vorschuß an Natriummethylat erhalten bleibt und am Schluß, wenn die Zugabe von Natriummethylat beendet ist, nur die Zugabe des restlichen Chlorbuttersäureesters erfolgt. Während der ganzen Zugabe von Chlorbuttersäureester destilliert ständig ein Gemisch von Methanol und Cyclopropancarbonsäuremethylester ab.
Das Molverhältnis Natriummethylat zum 4-Chlorbuttersäuremethylester beträgt 1 : 1 bis 2 : 1, vorzugsweise 1 : 1 bis 1,5 : 1, insbesondere 1,1 : 1 bis 1,2 : 1.
Das Gewichtsverhältnis Lösungsmittel zu eingesetztem 4-Chlorbuttersäureester beträgt 1 : 1 bis 1 : 2, vorzugsweise 1 : 1 bis 1 : 1,1.

Den erhaltenen Cyclopropancarbonsäureester setzt man beispielsweise ein, um durch Hydrierung das Hydroxymethylcyclopropan (Cyclopropylmethanol) zu erhalten. Hydroxymethylcyclopropan ist ein wichtiges Zwischenprodukt zur Herstellung von Pharma-Produkten. Die Hydrierung führt man bevorzugt in der Sumpf- und Rieselphase eines Zn-Chromit-Katalysators bei 200 bis 350 °C und 200 bis 320 bar Wasserstoffdruck durch.

Den folgenden Beispielen ist weiteres Zahlenmaterial zu entnehmen, sie sollen das erfindungsgemäße Verfahren näher erläutern.

### Beispiel 1

Man benutzt eine Glasapparatur, die aus einem Dreihalskolben mit Rührer, Thermometer und 10 cm langer Vigreux-Kolonne mit Destillationsbrücke besteht und an die zwei Dosierpumpen mit Vorlagen angeschlossen sind.

Man setzt ein:
500 g n-C₁₀-C₁₃-Alkylbenzol (Handelsname: MARLICAN)
und erwärmt auf 134 °C.

Dann werden in einer halben Stunde 200 ml Natriummethylatlösung (30 %ig) zugegeben und das Methanol abdestilliert. Danach werden bei 130 °C in jeweils einer halben Stunde gleichmäßig 80 ml Natriummethylatlösung 30 %ig, insgesamt 800 ml = 810 g (4,50 Mol) und 50 ml 4-Chlorbuttersäuremethylester, insgesamt 485 ml = 557 9 (4,07 Mol), zugepumpt. Hierbei destilliert ein Methanol-Ester-Gemisch ab. Die Kopftemperatur steigt von 66 auf 76 °C und fällt gegen Ende der Reaktion auf 66 °C ab.
Nach 5,5 Stunden ist die Zugabe des Chlorbuttersäureesters beendet. Dann wird noch eine halbe Stunde bei 130 °C gerührt und auf 80 °C abgekühlt. Um den restlichen Cyclopropancarbonsäuremethylester aus dem Sumpf zu destillieren, wird ein Unterdruck von 100 mbar angelegt. In einem Siedebereich von 49 bis 33 °C fallen 123 g Destillat an.
Destillatanfall insgesamt: 1 059 g
Der Chlorgehalt liegt bei 300 ppm.

Dieser Anfall wird an einer 0,5 m langen, mit Multifil-Füllkörpern gefüllten Kolonne bei Normaldruck fraktioniert destilliert.
Bei 117 °C fallen 359 g Cyclopropancarbonsäuremethylester mit einer Reinheit von 99,9 % und einem Chlorgehalt von nur 3,5 ppm an. Die Ausbeute beträgt 88,0 % der Theorie, bezogen auf Einsatz.

### Beispiele 2 und 3

Man verwendet die im Beispiel 1 beschriebene Apparatur und setzt die hier beschriebenen Produkte in den angegebenen Mengen ein mit dem Unterschied, daß die Natriummethylatlösung und der 4-Chlorbuttersäuremethylester gleichzeitig in den Reaktor gepumpt werden. Die weitere Durchführung erfolgt wie im Beispiel 1 beschrieben. Der in einer Ausbeute von 85,6 % erhaltene Cyclopropancarbonsäuremethylester hat einen Chlorgehalt von 190 ppm.
Die Wiederholung dieses Versuches liefert den gewünschten Ester in 83,8 %iger Ausbeute mit einem Chlorgehalt von 80 ppm., d. h. der Chlorgehalt bei dieser Arbeitsweise schwankt und ist viel zu hoch.

### Beispiel 4

Man benutzt die im Beispiel 1 beschriebene Apparatur, setzt die hier genannten Produkte in der hier beschriebenen Art ein, mit dem Unterschied, daß man als Reaktionstemperatur nicht 130 °C, sondern 110 °C wählt. Bei sonst gleicher Durchführung steigt der Reaktorinhalt immer weiter, weil zu wenig Produkt abdestilliert. Die Ausbeute liegt nach Aufarbeitung bei 87,6 % und der Chlorgehalt des Zielproduktes bei 7 ppm.

### Beispiel 5

Man arbeitet wie im Beispiel 4 beschrieben, nur mit dem Unterschied, daß man eine Reaktionstemperatur von 140 °C einstellt. Bei dieser Temperatur tritt starke Schaumbildung auf, und es besteht die Gefahr, daß Produktschaum mit dem Destillat übergeht. Nach der Aufarbeitung liegt die Ausbeute bei 87,9 % und der Chlorgehalt bei 10 ppm.

### Beispiele 6 und 7

Man arbeitet wie im Beispiel 1 beschrieben, mit dem Unterschied, daß man die Hälfte der einzusetzenden Natriummethylatlösung als Vorschuß einsetzt. Es entsteht eine rührfähige, aber erheblich dickflüssigere Suspension als beim Beispiel 1. Die Ergebnisse sind mit denen des Beispiels 1 vergleichbar.
Setzt man nur 5 % der Natriummethylatlösung als Vorschuß ein, ist die Suspension sehr dünnflüssig und man erhält ebenfalls wieder vergleichbare Ergebnisse wie beim Beispiel 1.

## Patentansprüche

1. Verfahren zur Herstellung von chlorfreiem Cyclopropancarbonsäuremethylester durch Umsetzung von 4-Chlorbuttersäuremethylester mit Natriummethylat in einem inerten Lösungsmittel,
dadurch gekennzeichnet,
daß man
a) zunächst das Lösungsmittel vorlegt, auf 100 bis 200 °C erwärmt,
b) einen Teil des einzusetzenden Alkalimethylats zugibt,
c) erst danach weiteres Alkalimethylat und 4-Chlorbuttersäuremethylester gleichzeitig in stöchiometrischem Verhältnis zugibt,
d) ständig ein Gemisch von Methanol und Cyclopropancarbonsäuremethylester abdestilliert und e) anschließend dieses Gemisch durch fraktionierte Destillation reinigt.

2. Verfahren nach Anspruch 1,
dadurch gekennzeichnet,
daß man die Umsetzung bei 100 bis 150 °C, vorzugsweise bei 120 bis 140 °C, durchführt.

3. Verfahren nach den Ansprüchen 1 und 2,
dadurch gekennzeichnet,
daß man ein hochsiedendes inertes Lösungsmittel verwendet, dessen Siedepunkt über 150 °C, vorzugsweise über 250 °C, insbesondere über 280 °C, liegt.

4. Verfahren nach den Ansprüchen 1 bis 3,
dadurch gekennzeichnet,
daß man als Vorschuß an Alkalimethylat 50 bis 5 %, vorzugsweise 10 bis 30 %, insbesondere 20 bis 25 %, der Gesamtmenge vorlegt.

5. Verfahren nach den Ansprüchen 1 bis 4,
dadurch gekennzeichnet,
daß man laufend während der Umsetzung ein Gemisch aus Methanol und Cyclopropancarbonsäuremethylester abdestilliert und am Schluß der Umsetzung Vakuum anlegt und restlichen Cyclopropancarbonsäuremethylester aus dem Lösungsmittel heraus überdestilliert.

6. Verfahren nach den Ansprüchen 1 bis 5,
dadurch gekennzeichnet,
daß man bei einem Molverhältnis Alkalimethylat zu 4-Chlorbuttersäuremethylester von 1,1 : 1 bis 1,2 : 1 cyclisiert.

7. Verfahren nach den Ansprüchen 1 bis 6,
dadurch gekennzeichnet,
daß man als Alkalimethylat Natriummethylat einsetzt.

8. Verfahren nach den Ansprüchen 1 bis 7,
dadurch gekennzeichnet,
daß man das erhaltene Methanol-Cyclopropancarbonsäuremethylester-Gemisch mittels fraktionierter Destillation reinigt.

## Claims

1. A process for the preparation of chlorine-free methyl cyclopropanecarboxylate by reaction of methyl 4-chlorobutyrate with sodium methoxide in an inert solvent, characterized in that
a) the solvent is first introduced, heated to from 100 to 200°C,
b) a portion of the alkali metal methoxide to be used is added,
c) only thereafter, further alkali metal methoxide and methyl 4-chlorobutyrate are simultaneously added in the stoichiometric ratio,
d) a mixture of methanol and methyl cyclopropanecarboxylate is continuously distilled off and
e) this mixture is then purified by fractional distillation.

2. A process according to claim 1, characterized in that the reaction is carried out at from 100 to 150°C, preferably at from 120 to 140°C.

3. A process according to either of claims 1 and 2, characterized in that a high-boiling inert solvent is used whose boiling point is above 150°C, preferably above 250°C, in particular above 280°C.

4. A process according to any of claims 1 to 3, characterized in that the amount of alkali metal methoxide introduced in advance is from 50 to 5%, preferably from 10 to 30%, in particular from 20 to 25%, of the total amount.

5. A process according to any of claims 1 to 4, characterized in that, during the reaction, a mixture of methanol and methyl cyclopropanecarboxylate is constantly distilled off and, at the end of the reaction, vacuum is applied and residual methyl cyclopropane carboxylate is distilled over from the solvent.

6. A process according to any of claims 1 to 5, characterized in that cyclization is performed at a molar ratio of alkali metal methoxide to methyl 4-chlorobutyrate of from 1.1:1 to 1.2:1.

7. A process according to any of claims 1 to 6, characterized in that the alkali metal methoxide used is sodium methoxide.

8. A process according to any of claims 1 to 7, characterized in that the methanol/methyl cyclopropane carboxylate mixture obtained is purified by fractional distillation.

## Revendications

1. Procédé pour préparer un cyclopropanecarboxylate de méthyle sans chlore, par réaction de 4-chlorobutyrate de méthyle avec du méthylate de sodium dans un solvant inerte caractérisé en ce que
a) on met en place d'abord le solvant, on le chauffe à une température de 100 à 200°C
b) on ajoute une partie du méthylate d'un métal alcalin à utiliser,
c) seulement ensuite, on ajoute le reste du méthylate d'un métal alcalin et le 4-chlorobutyrate de méthyle, simultanément selon des proportions stoechiométriques,
d) on chasse en continu par distillation un mélange de méthanol et de cyclopropanecarboxylate de méthyle, puis
e) on purifie ce mélange par distillation fractionnée.

2. Procédé selon la revendication 1, caractérisé en ce qu'on met en oeuvre la réaction à une température de 100 à 150°C et de préférence de 120 à 140°C.

3. Procédé selon les revendications 1 et 2, caractérisé en ce qu'on utilise un solvant inerte à haut point d'ébullition, dont le point d'ébullition est supérieur à 150°C, de préférence supérieur à 250°C, et en particulier supérieur à 280°C.

4. Procédé selon les revendications 1 à 3, caractérisé en ce qu'on met en place au départ une quantité de méthylate d'un métal alcalin de 50 à 5 %, de préférence de 10 à 30 % et en particulier de 20 à 25 % de la quantité totale.

5. Procédé selon les revendications 1 à 4, caractérisé en ce qu'on chasse en continu par distillation, pendant la réaction, un mélange de méthanol et de cyclopropanecarboxylate de méthyle, et, à la fin de la réaction, on applique un vide, le cyclopropanecarboxylate de méthyle restant étant extrait du solvant par distillation.

6. Procédé selon les revendications 1 à 5, caractérisé en ce qu'on procède à la cyclisation en présence d'un rapport en moles du méthylate d'un métal alcalin au 4-chlorobutyrate de méthyle de 1,1 : 1 à 1,2 : 1.

7. Procédé selon les revendications 1 à 6, caractérisé en ce qu'on utilise comme méthylate d'un métal alcalin le méthylate de sodium.

8. Procédé selon les revendications 1 à 7, caractérisé en ce qu'on purifie par distillation fractionnée le mélange obtenu de méthanol et de cyclopropanecarboxylate de méthyle.
